# EUROPÄISCHE PATENTSCHRIFT

(11) **EP 0 998 215 B1**
(45) Veröffentlichungstag und Bekanntmachung des Hinweises auf die Patenterteilung: **21.01.2004**
(21) Anmeldenummer: 98943652.2
(22) Anmeldetag: 08.07.1998
(51) Int. Cl.: A61B 5/00, G01N 21/49

(54) **VERFAHREN ZUM AUFFINDEN EINES UNTERSUCHUNGSORTES ZUR DIAPHANOSKOPISCHEN UNTERSUCHUNG EINES LEBEWESENS SOWIE VORRICHTUNG ZUR DURCHFÜHRUNG DES VERFAHRENS**
METHOD FOR DETERMINING AN EXAMINATION POINT FOR THE DIAPHANOSCOPIC EXAMINATION OF A BEING AND DEVICE FOR REALISING THE SAME
PROCEDE DE DETECTION D'UN POINT D'EXAMEN POUR L'EXAMEN DIAPHANOSCOPIQUE D'UN ETRE VIVANT ET DISPOSITIF POUR METTRE EN OEUVRE LEDIT PROCEDE

(30) Priorität: 21.07.1997 DE 19731254
(43) Veröffentlichungstag der Anmeldung: 10.05.2000
(73) Patentinhaber: SIEMENS AKTIENGESELLSCHAFT, 80333 München (DE)
(72) Erfinder: ABRAHAM-FUCHS, Klaus, D-91058 Erlangen (DE); BEUTHAN, Jürgen, D-12165 Berlin (DE); PRAPAVAT, Viravuth, D-22081 Hamburg (DE); MÜLLER, Gerhard, D-14129 Berlin (DE)
(86) Internationale Anmeldenummer: PCT/DE1998/001886
(87) Internationale Veröffentlichungsnummer: WO 1999/004684

(56) Entgegenhaltungen:
- EP-A- 0 710 832
- BEUTHAN J ET AL: "OPTISCHE DIFFUSIONSTOMOGRAPHIE ZUR MESSUNG OPTISCHER GEWEBEPARAMETER IN DER RHEUMADIAGNOSTIK OPTICA DIFFUSION TOMOGRAPHY FOR MEASUREMENT OF OPTICAL TISSUE PARAMETERS IN DIAGNOSIS OF RHEUMATISM" TECHNISCHES MESSEN TM, Bd. 63, Nr. 6, Juni 1996, Seiten 234-240, XP000620446
- SHANTHI S ET AL: "LASER REFLECTANCE IMAGING OF HUMAN ORGANS AND COMPARISON WITH PERFUSION IMAGES" MEDICAL AND BIOLOGICAL ENGINEERING AND COMPUTING, Bd. 35, Nr. 3, Mai 1997, Seiten 253-258, XP000677434

## Beschreibung

Die Erfindung betrifft ein Verfahren zum Auffinden eines Untersuchungsortes zur diaphanoskopischen Untersuchung eines Lebewesens.

Im Rahmen diaphanoskopischer Untersuchungen wird ein zu untersuchender Bereich eines Lebewesens mit Licht durchleuchtet und das aufgenommene Durchleuchtungsbild zur Diagnose herangezogen. Möchte man nun eine pathologisch induzierte optische Änderung in einem bestimmten durchstrahlten Volumen des Lebewesens detektieren und das Ergebnis zur Grundlage der Diagnose zur Verfügung stellen, so ist durch die Auswahl eines "optimalen" Einstrahlortes sicherzustellen, daß das von den Photonen durchdrungene pathologisch veränderliche Gewebe im Gegensatz zum pathologisch konstanten Volumen maximal ist, um auf diese Weise im Rahmen der Hauptuntersuchung tatsächlich den Bereich maximaler Information untersuchen zu können. Dies ist insbesondere von Bedeutung, wenn die zu detektierende pathologische Änderung klein ist. Ein diaphanoskopisches Untersuchungsverfahren kann beispielsweise an einem Fingergelenk durchgeführt werden, um Untersuchungen betreffend rheumatoider Arthritis durchzuführen. Vereinfacht setzt sich ein Fingergelenk aus Knochen-, Knorpel-, Haut- und Kapselgewebe sowie Gelenkflüssigkeit zusammen. Dabei bleiben das Knochengewebe, Knorpelgewebe sowie das umhüllende Hautgewebe im Frühstadium pathologisch konstant, die möglichen pathologischen Veränderungen treten allein in der Gelenkkapsel sowie der Gelenkflüssigkeit auf. Um bezüglich dieses relativ schmalen aussagekräftigen Volumens einen maximalen Informationsgehalt im Rahmen der Hauptuntersuchung erhalten zu können, ist es erforderlich, die Durchleuchtung am optimalen Untersuchungsort durchzuführen, so daß der erhaltene Informationsgehalt größtmöglich ist. Als Untersuchungsort wird dabei der durch einen Positionswert angebbare Gewebeort verstanden, der vom Strahlquerschnitt der zur Untersuchung verwendeten Lichtquelle beleuchtet wird.

Aus US-PS 5452723 ist ein Spektroskopieverfahren bekannt, welches im Rahmen der Spektroskopie menschlichen Gewebes zum Einsatz kommt. Mit dem dort beschriebenen Verfahren sollen die sich bei einer Untersuchung eines dicken, mehrere Millimeter starken Gewebes ergebenden Verzerrungen der erhaltenen Meßwerte bedingt durch die erhöhte Anzahl an Streuzentren des dicken Gewebes, im Vergleich zur Spektroskopie eines sehr dünnen, nur wenige Mikrometer dicken Gewebes, bei dem weniger das Meßergebnis beeinflussende Streuzentren gegeben sind, kompensiert werden. Dies erfolgt dergestalt, daß zunächst ein Spektrum der diffusen Reflektanz aufgenommen wird, anschließend das zu "entzerrende" Spektrum, beispielsweise das Fluoreszenzspektrum. Anschließend wird unter Zugrundelegung von Wahrscheinlichkeitsfunktionen eine effektive Reflektanzfunktion ermittelt. Das entzerrte Fluoreszenzspektrum wird dann durch Division des aufgenommen Fluoreszenzspektrums durch das anhand der effektiven Reflektanzfunktion beschriebenen effektiven Reflektanzspektrums ermittelt. Die sich aus Streu- und Absorbtionseffekten sowie der Geometrie- und den Grenzflächenbedingungen ergebenden Verzerrungen des Spektrums des dicken Gewebes werden eliminiert, der erhaltene Spektrumsverlauf entspricht in guter Näherung dem eines dünnen Gewebes. Anschließend wird die erhaltene "entzerrte" Meßkurve mit bekannten Referenzkurven verglichen und die beste Fitkurve bestimmt, welche anschließend hinsichtlich der Anwesenheit und Konzentration von Referenzfluoroforen untersucht wird, was Grundlage für die Diagnose der entsprechende Gewebeeigenschaft ist.

Aus BEUTHAN J. et al: "Optische Diffusionstomographie zur Messung optischer Gewebeparameter in der Rheumadiagnostik"; TECHNISCHES MESSEN TM , Band 63, Nr. 6, 1996, Seiten 234 ff, ist ein Verfahren zur diaphanoskopischen Untersuchung eines Lebewesens bekannt, bei dem ein Bereich des Lebewesens mit einer Strahlung, vorzugsweise in einem Wellelängenbereich des optischen Gewebefensters, zur Aufnahme von Streulichtverteilungen durchleuchtet wird.

Der Erfindung liegt damit das Problem zugrunde, ein Verfahren anzugeben, mittels welchem der optimale Untersuchungsort für derartige diaphanoskopische Untersuchungen beispielsweise von Gelenken ermittelt werden kann.

Zur Lösung dieses Problems ist ein Verfahren vorgesehen, bei dem ein Bereich des Lebewesens, in dem der optimale Untersuchungsort vermutet wird, sequentiell mit einer Strahlung vorzugsweise in einem Wellenlängenbereich des optischen Gewebefensters zur Aufnahme von Streulichtverteilungen in Form von ortsbezogenen Verwaschungsfunktionen, insbesondere Punktverwaschungsfunktionen durchleuchtet wird, wobei wenigstens ein funktionsspezifisches ortsbezogenes Merkmal jeder Verwaschungsfunktion ermittelt wird, auf welchen basierend ein den Untersuchungsort definierender Positionswert ermittelt wird.

Das erfindungsgemäße Verfahren beruht darauf, daß bei einer (punktförmigen) ortsabhängigen Durchleuchtung des zu untersuchenden Gewebes Streulichtverteilungen in Form von Verwaschungsfunktionen (bei punktförmiger Einstrahlung von Punktverwaschungsfunktionen) entstehen, deren Änderung in Form und Skalierung über den Einstrahlort eine Funktion der Lichtausbreitung sind, und die somit eine Aussage über die optischen Verhältnisse des durchleuchteten Gesamtvolumens ermöglichen. Bei Kenntnis der optischen Eigenschaften der im durchleuchteten Volumen vorhandenen Gewebearten kann dann ermittelt werden, in welcher Art und Weise sich die Photonen ausbreiten, wenn diese hauptsächlich pathologisch veränderliches Volumen durchdringen, und in welcher Form- und Skalierungsänderung sich dies in der resultierenden Streulichtverteilung bzw. Verwaschungsfunktion darstellt. Erfindungsgemäß werden also verschiedene, beispielsweise fünf, Verwaschungsfunktionen an unterschiedlichen Einstrahlorten aufgenommen und aufgrund der jeweils erhaltenen Verwaschungsfunktion, wonach zu jeder ein funktionsspezifisches ortsbezogenes Merkmal, also ein aussagekräftiges Kennzeichen jeder Funktion ermittelt wird. Nach Bestimmung dieser Merkmale werden diese gemeinsam weiterverarbeitet und basierend hierauf ein Positionswert ermittelt, welcher den optimalen Untersuchungsort definiert, das heißt, auf dem basierend der optimale Untersuchungsort bestimmt werden kann.

Zur Durchführung wird ein Bereich des Lebewesens, der den optimalen Untersuchungsort als Teilbereich umfaßt, an mehreren Orten des Bereichs zeitlich aufeinanderfolgend durchleuchtet. Dies kann z. B. in äquidistanten Schritten entlang einer Linie, etwa parallel zur Längsachse eines Fingers (im folgenden x-Achse genannt), erfolgen. Es wird vorzugsweise eine Strahlung im Wellenlängenbereich des optischen Gewebefensters verwendet. Bei Durchdringung des Gewebes wird das (näherungsweise) punktförmig eingestrahlte Licht gestreut. Die räumliche Verteilung des Streulichts wird mit einer flächenhaften oder linienförmigen Anordnung von Lichtdetektoren erfaßt. Die so gemessene Intensität des Streulichts als Funktion des Ortes der Lichtdetektoren wird als Streulichtverteilungsfunktion, oder spezifischer auch als (Punkt-) Verwaschungsfunktion, bezeichnet. Zur näheren Erläuterung des Begriffs "Verwaschungsfunktion" wird z. B. auf Eugene Hecht, "Optik", Addison-Wesley-Verlag, 1989, S. 512 ff. verwiesen. Die Verlaufsform dieser Verwaschungsfunktion ist abhängig von der Zusammensetzung des durchstrahlten Gewebes, und somit abhängig vom gewählten Einstrahlort. Man erhält also für jeden sequentiell gewählten Einstrahlort eine eigene charakteristische Verwaschungsfunktion. Allgemein können Funktionsverläufe durch einen oder mehrere charakteristische Werte (funktionspezifische Parameter oder Merkmale) beschrieben werden ("Parametrisieren" einer Funktion), z. B. Maximalwert der Funktion, Ort des Maximalwertes der Funktion, Ort der maximalen Steigung oder Krümmung der Funktion etc. Für das hier vorgeschlagene Verfahren wird mindestens ein oder eine mathematische Verknüpfung von mehreren Beschreibungs-Parametern der Funktion ausgewählt, der charakteristisch für die Veränderung der Verwaschungsfunktion bei Variation des Einstrahlortes, insbesondere für die Unterschiede der Form der Verwaschungsfunktion bei Durchstrahlung von gesundem und erkranktem Gewebe, ist. Diese Parameter zur Beschreibung des Funktionsverlaufs der gemessenen Verwaschungsfunktionen werden im folgenden als funktionsspezifische orts- (auf den Ort der Einstrahlung) bezogene Merkmale bezeichnet. Der Vergleich der Werte dieser Parameter ermöglicht nun die Auswahl derjenigen Verwaschungsfunktion, und somit die Auswahl desjenigen Einstrahlortes oder optimalen Untersuchungsortes, der am meisten Informationen über die pathologische Veränderung des Gewebes enthält.

Als ortsbezogene funktionsspezifische Merkmale können beispielsweise Ortsparameter, also x-Werte der Verwaschungsfunktion bezüglich ausgezeichneter Punkte der Verwaschungsfunktion bestimmt und anschließend weiterverarbeitet werden.

Als Merkmale können erfindungsgemäß z. B. die jeweiligen Positionswerte, also die x-Werte der Maxima und/oder der Schwerpunkte der Verwaschungsfunktionen verwendet werden. Dabei kann erfindungsgemäß der den Untersuchungsort definierende Positionswert durch Mittelwertbildung aus den Positionswerten der Maxima oder der Schwerpunkte ermittelt werden. Wie sich im Rahmen von Versuchen gezeigt hat, sind die erhaltenen Streulichtverteilungen unterschiedlich aussagekräftig, je nachdem, wie nahe der jeweilige Einstrahlort zum optimalen Untersuchungsort liegt. Unter Berücksichtigung der optischen Eigenschaften dieser Gewebe kann angenommen werden, daß mit hoher Wahrscheinlichkeit ein geeigneter Einstrahlort dort zu finden ist, wo die Gesamtbestrahlungsstärke (Fläche der resultierenden Streulichtverteilung) groß und die Breite der resultierenden Streulichtverteilung schmaler gegenüber den Verteilungen ist, die bei Einstrahlung um diesen Punkt bestehen. Der Informationsgehalt bezüglich der einzelnen Verwaschungsfunktionen hinsichtlich der Ermittlung des Untersuchungsortes ist damit jeweils unterschiedlich. Um dem zu begegnen sieht eine zweckmäßige Weiterführung des Erfindungsgedankens vor, daß jedes funktionsspezifische ortsbezogene Merkmal mit einem Wichtungsfaktor gewichtet wird, welcher zweckmäßigerweise basierend auf wenigstens einem, gegebenenfalls weiteren funktionsspezifischen Merkmal bestimmt werden kann. Durch Berücksichtigung dieses Wichtungsfaktors trägt also jede Verwaschungsfunktion unterschiedlich zum ermittelten Positionswert bei, das heißt, die näher oder am optimalen Untersuchungsort liegende Funktion geht wesentlich stärker ein als die danebenliegenden. Der Wichtungsfaktor kann zweckmäßigerweise multiplikativ aus der Gesamtbestrahlungsstärke und der Standardabweichung jeder Verwaschungsfunktion bestimmt werden, da die Wahrscheinlichkeit groß ist, einen geeigneten Einstrahlort dort zu finden, an dem die Gesamtbestrahlungsstärke groß und die Breite der Streulichtverteilung klein ist gegenüber Verteilungen um diesen Punkt. Die Wichtungsfaktoren können ferner auf den größten ermittelten Wichtungsfaktor normiert werden.

Mit dem beschriebenen Ermittlungsverfahren des Positionswerts durch Mittelwertbildung, gegebenenfalls durch gewichtete Mittelwertbildung kann der optimale Untersuchungsort hinreichend genau bestimmt werden, um eine aussagekräftige Untersuchung durchführen zu können. Jedoch erfolgt die Ermittlung dieses Untersuchungsortes an der Detektorseite, da die erhaltenen Verwaschungsfunktionen detektorseitig bestimmt werden und die erhaltenen Positionswerte aus diesen detektorseitigen Informationen gewonnen werden. Für den Fall, daß bedingt durch die physiologischen Gegebenheiten des durchleuchteten Volumens die Einstrahlorte deutlich von den ermittelten ortsbezogenen Positionswerten aus den Verwaschungsfunktionen abweichen, kann der quasi detektorseitig ermittelte Positionswert zu ungenau sein, um ihn unmittelbar auf die Einstrahlebene zu übertragen. Dies kann beispielsweise bei einem Fingergelenk dadurch hervorgerufen werden, daß das Kapselgewebe und die Gelenkflüssigkeit lichtleitende Eigenschaften haben und so hier eingekoppeltes Licht bevorzugt an einer bestimmten Position wieder ausgekoppelt wird. Um dem zu begegnen kann gemäß einer vorteilhaften weiteren Erfindungsausgestaltung vorgesehen sein, daß nach Ermittlung des Positionswertes eine Rücktransformation in die Einstrahlebene erfolgt. Dies kann zweckmäßigerweise dadurch realisiert werden, daß zur Rücktransformation ein weiterer Positionswert ermittelt wird, um welchen der ermittelte erste, sich auf die Detektorseite beziehende Positionswert verschoben wird. Das heißt, als weiterer Positionswert wird ein Korrekturwert ermittelt, der den durch die physiologischen Gegebenheiten hervorgerufenen Versatz zwischen dem Einstrahlort und dem bestimmten ersten Positionswert kompensiert und welcher zur entsprechenden Verschiebung des ermittelten ersten Positionswertes verwendet wird, so daß der hierdurch erhaltene Positionswert den Einstrahlort in der Einstrahlebene kennzeichnet. Dabei kann erfindungsgemäß als weiterer Positionswert der Versatz zwischen dem einstrahlseitigen Einstrahlort der Verwaschungsfunktion mit der größten Gesamtbestrahlungsstärke oder dem größten Wichtungsfaktor und der detektionsseitigen Lage des Funktionsmaximums oder des Schwerpunkts dieser Verwaschungsfunktion verwendet werden.

Der oder die Positionsdaten können erfindungsgemäß anhand der unmittelbar erhaltenen Verwaschungsfunktionen oder einer zumindest im Bereich der Maxima geglätteten Verwaschungsfunktion ermittelt werden, sofern der Kurvenverlauf eine entsprechende, einen akzeptierbaren Fehler aufweisende Ermittlung zuläßt. Wenn dies nicht mit einem akzeptablen Fehler möglich ist, sieht eine zweckmäßige Weiterbildung des Erfindungsgedankens vor, daß die erhaltenen Verwaschungsfunktionen zur Bestimmung von Approximationsfunktionen approximiert werden, wobei der oder die Positionswerte anhand der Approximationsfunktionen ermittelt werden. Zweckmäßigerweise kann jede Verwaschungsfunktion mittels einer oder mehrerer Gaußfunktionen approximiert werden.

Neben dem erfindungsgemäßen Verfahren betrifft die Erfindung ferner eine Vorrichtung zur Durchführung des Verfahrens. Diese Vorrichtung umfaßt ein Bestrahlungsmittel, ein Detektormittel, ein Steuermittel und ein Rechenmittel und zeichnet sich erfindungsgemäß dadurch aus, daß das Rechenmittel zum Ermitteln eines optimalen Untersuchungsortes nach dem erfindungsgemäßen Verfahren und das Steuerungsmittel zur Steuerung des Bestrahlungsmittels in Abhängigkeit des Ermittlungsergebnisses ausgebildet ist. Dabei kann erfindungsgemäß das Bestrahlungsmittel eine Strahlungsquelle für eine bestimmte Wellenlänge aufweisen und von dem Steuermittel steuerbare Mittel zum Bewegen der Strahlungsquelle vorgesehen sein. Bei dieser ersten Erfindungsausgestaltung kommt somit lediglich eine Strahlungsquelle zum Einsatz, welche nach Ermittlung des optimalen Untersuchungsortes zu diesem über die entsprechenden Bewegungsmittel gefahren wird. Alternativ hierzu kann das Bestrahlungsmittel auch mehrere mittels des Steuermittels separat ansteuerbare Strahlungsquellen für eine bestimmte Wellenlänge aufweisen. Bei diesem rasterartig ausgebildeten Bestrahlungsmittel ist die Bewegbarkeit nicht unbedingt erforderlich, insbesondere dann nicht, wenn das Rastermaß, also der Abstand der Strahlungsquellen zueinander, hinreichend klein gewählt wird. Für den Fall, daß das Rastermaß relativ grob ist, können erfindungsgemäß über die Steuermittel in Abhängigkeit des Ermittlungsergebnisses steuerbare Mittel zum Bewegen wenigstens einer, vorzugsweise aller Strahlungsquellen vorgesehen sein. Ein möglicher Betrieb ohne Bewegungsmittel kann beispielsweise derart sein, daß das Rastermaß der Strahlungsquellen sehr klein ist und zur Ermittlung des Untersuchungsortes nur bestimmte, einen gröberen Abstand aufweisende Strahlungsquellen angesteuert werden. Nach Ermittlung des optimalen Untersuchungsortes kann dann eine andere Strahlungsquelle, die optimiert zum optimalen Untersuchungsort infolge des sehr kleinen Rasters liegt, angesteuert werden. Das Rastermaß der Strahlungsquellen und der Durchmesser des jeweils emittierten Lichtstrahls sollte in einem Verhältnis von ungefähr 1:1 bis ungefähr 3:1, insbesondere 2:1 stehen. Für den Fall, daß die Strahlungsquellen bewegbar sind, hat sich ein Rastermaß der Strahlungsquellen von ungefähr 250 µm bis 750 µm, insbesondere von 500 µm als zweckmäßig herausgestellt, wobei hier der Strahldurchmesser ungefähr 125 µm bis 500 µm, insbesondere 250 µm betragen sollte. Bei unbeweglichen Strahlungsquellen sollte das Rastermaß möglichst klein sein, und in einem Bereich von ungefähr 125 µm bis 500 µm, insbesondere bei 250 µm liegen. Der Strahldurchmesser kann auch hier zwischen 125 µm und 500 µm, insbesondere 250 µm betragen. Um einerseits die Auffindung des optimalen Untersuchungsortes und andererseits die Durchführung der Untersuchung bei mehreren Wellenlängen durchführen zu können, was mithin zu aussagekräftigeren Ergebnissen führt, kann erfindungsgemäß ferner vorgesehen sein, daß Strahlungsquellen für mehrere Wellenlängen vorgesehen sind.

Weitere Vorteile, Merkmale und Einzelheiten der Erfindung ergeben sich aus den im folgenden beschriebenen Ausführungsbeispielen sowie anhand der Zeichnungen. Dabei zeigen:
- Fig. 1: ein Diagramm mit fünf erhaltenen Streulichtverteilungen,
- Fig. 2: fünf zu den Streulichtverteilungen aus Fig. 1 errechneten Approximationskurven sowie die diesbezüglichen Schwerpunkte,
- Fig. 3: eine Prinzipskizze einer Anordnung einer ersten Ausführungsform, und
- Fig. 4: eine Prinzipskizze einer Anordnung einer zweiten Ausführungsform.

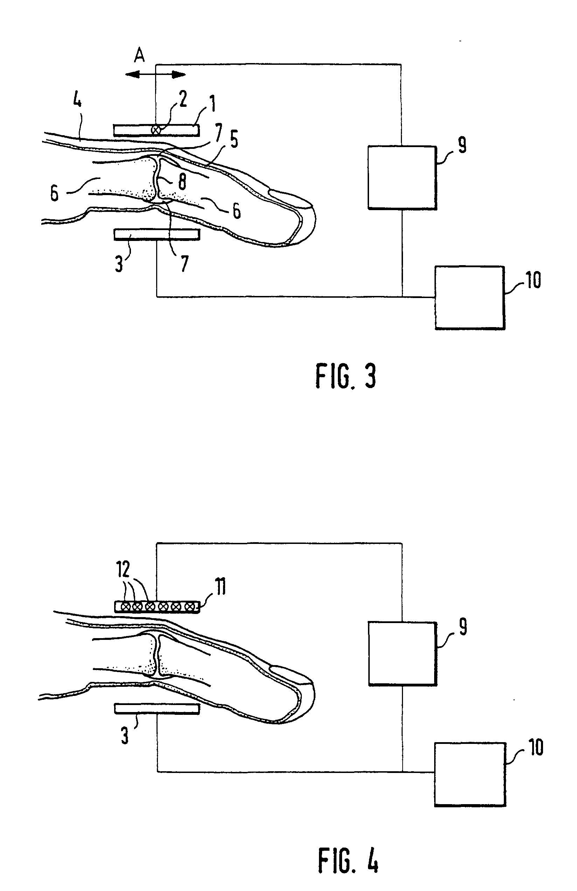

Zum besseren Verständnis des nachfolgend an einem Ausführungsbeispiel beschriebenen Ermittlungsverfahrens sei zunächst die erfindungsgemäße Vorrichtung zur Durchführung dieses Verfahrens beschrieben, wie beispielsweise in Fig. 3 gezeigt. Diese Vorrichtung besteht aus einem Bestrahlungsmittel 1 mit einer Strahlungsquelle 2 beispielsweise in Form eines Lasers mit einer Wellenlänge von 675 nm. Gegenüberliegend ist ein Detektormittel 3 vorgesehen. Zwischen Bestrahlungsmittel 1 und Detektormittel 3 wird der zu untersuchende Bereich eines Lebewesens, im gezeigten Beispiel ein Finger 4 gebracht.

Dieser Finger 4 besteht im zu untersuchenden Bereich eines Gelenks aus Hautgewebe 5, Knorpel- und Knochengewebe 6 sowie Gelenkkapselgewebe 7 und Gelenkflüssigkeit 8. Dem Beleuchtungsmittel und dem Detektormittel ist ein Steuerungsmittel 9 zum Steuern des Betriebs und ein Rechenmittel 10 zum Bestimmen des optimalen Untersuchungsortes zugeschaltet. Bei dieser nur eine Strahlungsquelle 2 aufweisenden Ausführungsform erfolgt nun der Betrieb zur Ermittlung des optimalen Untersuchungsortes derart, daß das Bestrahlungsmittel 1 mitsamt der Strahlungsquelle 2 an beispielsweise fünf ausgewählte Positionen oberhalb des Gelenkspaltes gefahren wird, wobei die Beweglichkeit durch den Doppelpfeil A angegeben ist. Die hierfür erforderlichen Bewegungsmittel sind nicht explizit dargestellt. An jedem der ausgezeichneten Orte erfolgt nun eine punktuelle Durchleuchtung des Gewebes. Mittels der Detektormittel 3 wird zu jeder Einstrahlung die jeweilige Streulichtverteilung in Form einer Punktverwaschungsfunktion aufgenommen und dem Rechenmittel 10 zugeführt, welches anhand des nachfolgend beschriebenen Verfahrens den optimalen Untersuchungsort betreffend die Einstrahlebene ermittelt und an die Steuermittel ausgibt, welche dann zur Durchführung der eigentlichen Untersuchung das Bestrahlungsmittel 1 entsprechend ansteuern und bewegen.

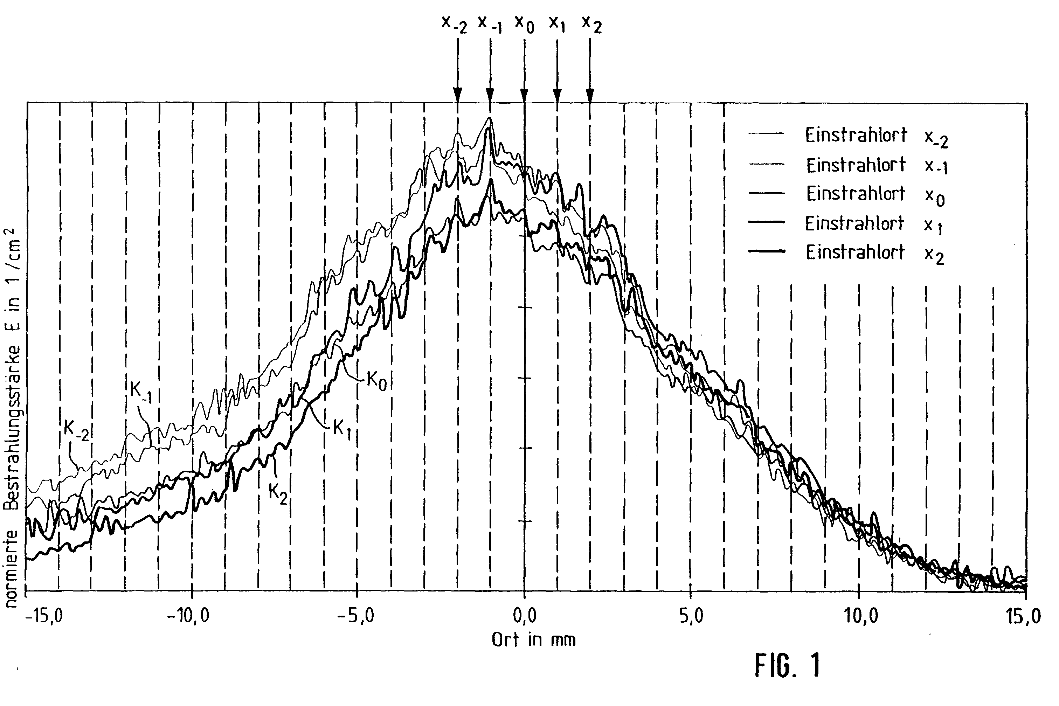

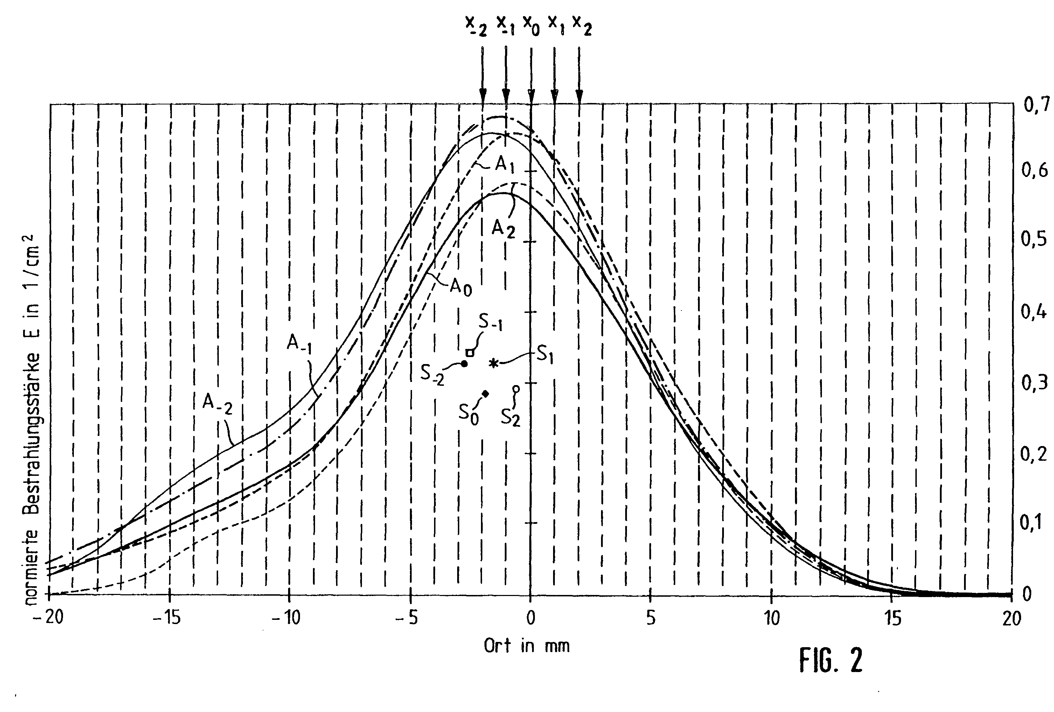

Wie bereits beschrieben, wird zu jedem der vorbestimmten Einstrahlorte um eine definierte Nullage die Streulichtverteilung aufgenommen. Derartige Streulichtverteilungen sind in Fig. 1 gezeigt, in der längs der Abszisse der Ort in mm um die Nullage und längs der Ordinate die normierte Bestrahlungsstärke in 1/cm² angegeben ist. Durch die Pfeile ist jeweils der Einstrahlort zu den jeweiligen Kurven angegeben. Die hier gezeigten Einstrahlorte liegen um ± 2 Millimeter um die Nullage herum verteilt, das heißt, insgesamt wurden fünf Einstrahlorte gewählt. Zu jedem der Einstrahlorte X₋₂ ... X₂ ist die jeweils erhaltene Streulichtverteilung mit den Bezugszeichen K₋₂...K₂ angegeben. Wie Fig. 1 zu entnehmen ist, ist der Kurvenverlauf doch relativ verrauscht und läßt nur eine ungenaue Bestimmung des optimalen Untersuchungsortes zu. Zu diesem Zweck wird zu jeder der Kurven K₋₂...K₊₂ eine Approximationskurve A₋₂...A₊₂ durch Approximation mittels mehrerer Gaußfunktionen ermittelt. Der Verlauf dieser Approximationskurven ist in Fig. 2 dargestellt. Darüber hinaus wurde der Vollständigkeit halber auch zu jeder Kurve A₋₂... A₊₂ der jeweilige Kurvenschwerpunkt S₋₂...S₊₂ ermittelt. Für die nachfolgend beschriebene Ermittlungsuntersuchungsorte werden die Schwerpunkte nicht herangezogen, jedoch könnte das Ermittlungsverfahren gleichermaßen basierend auf diesen Werten durchgeführt werden.

Zur Bestimmung des optimalen Untersuchungsortes werden nun eine Reihe von funktionsspezifischer ortsbezogener Merkmale bestimmt, wie sie aus der nachfolgenden Tabelle entnehmbar sind.

**Tabelle**

| Einstrahlort (in mm) | X₋₂=-2mm | X₋₁=1mm | X₀=0mm | X₁=1mm | X₂=2mm |
|---|---|---|---|---|---|
| x-Wert von Eₘₐₓ (in mm) | -1,6 | -1,4 | -1,2 | -0,6 | -0,8 |
| Gesamtbestrahlungsstärke E_{ges} | 50,0 | 50,8 | 42,3 | 47,0 | 39,7 |
| Standardabweichung s | 6,598 | 6,793 | 6,74 | 6,807 | 5,914 |
| Wichtungsfaktor W=E_{ges}•s | 329,9 | 345,08 | 285,13 | 319,93 | 234,8 |
| normierter Wichtungsfaktor | 0,956 | 1 | 0,826 | 0, 927 | 0, 680 |
| gewichteter x-Wert won Eₘₐₓ (in mm) | -1,53 | -1,4 | -0,99 | 0,56 | 0,54 |

Anhand der Approximationsfunktionen werden zunächst die x-Werte der jeweiligen Kurvenmaxima Eₘₐₓ bestimmt. Diese liegen, wie der Zeile 2 der Tabelle zu entnehmen ist, jeweils im negativen Bereich. Aus diesen Werten ist es bereits möglich, einen ersten Untersuchungsort durch Mittelwertbildung zu berechnen. Dieser läge bei x_{Mittel} = 1,12 mm.

Zur Berücksichtigung des tatsächlichen Beitrags der Streulichtverteilungen ist es zweckmäßig, einen Wichtungsfaktor zu ermitteln und bei der Bildung des Mittelwertes zu berücksichtigen. Zur Berechnung dieses Wichtungsfaktors bieten sich bestimmte Eigenschaften der Streulichtverteilung wie die Gesamtbestrahlungsstärke E_{ges} (= Fläche unter der jeweiligen Kurve) und die Standardabweichung (= Breite der Kurve) an. Die bezüglich der Approximationsfunktionen ermittelten Werte hierfür sind den Zeilen 3 und 4 zu entnehmen. Basierend hierauf ist es möglich, einen Wichtungsfaktor multiplikativ aus diesen beiden Werten zu ermitteln, wobei die Werte in Zeile 5 angegeben sind. Anschließend wird der ermittelte Wichtungsfaktor auf den höchsten Wichtungsfaktor normiert (Zeile 6). Werden nun die ermittelten x-Werte von Eₘₐₓ (Spalte 2) mit den normierten Wichtungsfaktoren multipliziert, so erhält man die gewichteten x-Werte von Eₘₐₓ, wie Zeile 7 angegeben. Der aus diesen gewichteten Werten errechenbare gewichtete Mittelwert beträgt x_{Mittel} = 1,004 mm und trägt dem jeweiligen Beitrag der einzelnen Streulichtverteilungen Rechnung. Im Vergleich zum ungewichtet ermittelten Positionswert ergibt sich somit eine Differenz von 0,116 mm.

Anschließend muß der gewichtete Mittelwert, der den optimalen Ort auf der Detektorseite spezifiziert, auf eine Koordinate der Anregungsseite rücktransformiert werden. Zu diesem Zweck wird der Versatz zwischen dem Einstrahlort, der die Streulichtverteilung mit dem größten Wichtungsfaktor bzw. dem größten E_{ges} liefert und der Lage von Eₘₐₓ dieser Streulichtverteilung verwendet. Im gezeigten Ausführungsbeispiel ist die Einstrahlposition, die die Streulichtverteilung mit dem größten Wichtungsfaktor liefert, der Einstrahlort X₋₁. Die Eₘₐₓ-Lage dieser Streulichtverteilung ist x_{Emax} = -1,4 mm. Hieraus ergibt sich ein Versatz von X₋₁ - x_{Emax} = -1 mm-(-1,4 mm) = +0,4 mm. Zur Rücktransformation wird nun der errechnete gewichtete Mittelwert von 1,004 mm um diesen Versatz verschoben, so daß die neue Nullage in der Einstrahlebene zu Xoptimal = -0,604 mm gewählt wird.

Das beschriebene Bestimmungsverfahren kann anstelle der Verwendung der x-Werte von Eₘₐₓ in gleicher Weise basierend auf den Schwerpunkten der jeweiligen Approximationsfunktionen durchgeführt werden.

Schließlich zeigt Fig. 4 eine weitere Ausführungsform einer erfindungsgemäßen Vorrichtung, die eine Ausführungsalternative zur aus Fig. 3 entnehmbaren Vorrichtung zeigt. Der Aufbau ist weitgehend der gleiche, jedoch kommt hier ein Bestrahlungsmittel 11 zum Einsatz, welches mehrere Strahlungsquellen 12 aufweist. Die Strahlungsquellen 12 sind rasterartig voneinander beabstandet, wobei bevorzugt ein Raster von 500 µm oder kleiner gewählt wird, bevorzugt 250 µm. Im gezeigten Ausführungsbeispiel sind der Übersichtlichkeit wegen nur sieben Strahlungsquellen angegeben, die Zahl ist jedoch selbstverständlich im Hinblick auf den gewählten Rasterabstand wesentlich größer. Die Betriebsweise dieser Vorrichtung ist nun derart, daß beispielsweise lediglich jede zweite Strahlungsquelle angesteuert wird, wobei natürlich nur die angesteuert werden, die in entsprechend naher Lage zum vermuteten optimalen Untersuchungsort liegen. Nach Ermittlung des optimalen Untersuchungsortes kann dann über das auch hier vorhandene Steuerungsmittel 9 eine günstigere weil näher zum Untersuchungsort liegende Strahlungsquelle angesteuert werden. Ein derartiger Betrieb ist bei hinreichend kleinem Abstand, beispielsweise von 250 µm, möglich. Sofern ein größerer Rasterabstand gewählt ist (beispielsweise 500 µm) ist es ebenfalls denkbar, eine gewisse Bewegbarkeit des Bestrahlungsmittels 11 zu realisieren, um eine ausgewählte Strahlungsquelle an den optimalen Untersuchungsort zu bringen.

## Patentansprüche

1. Verfahren zum Auffinden eines Untersuchungsortes zur diaphanoskopischen Untersuchung eines Lebewesens, bei dem ein Bereich des Lebewesens, in dem der optimale Untersuchungsort vermutet wird, sequentiell mit einer Strahlung vorzugsweise in einem Wellenlängenbereich des optischen Gewebefensters zur Aufnahme von Streulichtverteilungen in Form von ortsbezogenen Verwaschungsfunktionen, insbesondere Punktverwaschungsfunktionen durchleuchtet wird, wobei wenigstens ein funktionsspezifisches ortsbezogenes Merkmal jeder Verwaschungsfunktion ermittelt wird, auf welchen basierend ein den Untersuchungsort definierender Positionswert ermittelt wird.

2. Verfahren nach Anspruch 1, **dadurch gekennzeichnet, daß** als Merkmal die jeweiligen Positionswerte der Maxima und/oder der Schwerpunkte der Verwaschungsfunktionen verwendet werden.

3. Verfahren nach Anspruch 2, **dadurch gekennzeichnet, daß** der den Untersuchungsort definierende Positionswert durch Mittelwertbildung aus den Positionswerten der Maxima oder der Schwerpunkte ermittelt wird.

4. Verfahren nach einem der vorangehenden Ansprüche, **da-durch gekennzeichnet, daß** jedes funktionsspezifische ortsbezogene Merkmal mit einem Wichtungsfaktor gewichtet wird.

5. Verfahren nach Anspruch 4, **dadurch gekennzeichnet, daß** jeder Wichtungsfaktor basierend auf wenigstens einem, gegebenenfalls weiteren funktionsspezifischen Merkmal bestimmt wird.

6. Verfahren nach Anspruch 5, **dadurch gekennzeichnet, daß** jeder Wichtungsfaktor multiplikativ aus der Gesamtstrahlungsdichte und der Standardabweichung jeder Verwaschungsfunktion bestimmt wird.

7. Verfahren nach einem der Ansprüche 4 bis 6, **dadurch gekennzeichnet, daß** die Wichtungsfaktoren auf den größten ermittelten Wichtungsfaktor normiert werden.

8. Verfahren nach einem der vorangehenden Ansprüche, **dadurch gekennzeichnet, daß** nach Ermittlung des Positionswertes eine Rücktransformation in die Einstrahlebene erfolgt.

9. Verfahren nach Anspruch 8, **dadurch gekennzeichnet, daß** zur Rücktransformation ein weiterer Positionswert ermittelt wird, um welchen der ermittelte erste, sich auf die Detektionsebene beziehende Positionswert verschoben wird.

10. Verfahren nach Anspruch 9, **dadurch gekennzeichnet, daß** als weiterer Positionswert der Versatz zwischen dem einstrahlseitigen Einstrahlort der Verwaschungsfunktion mit der größten Gesamtbestrahlungsstärke oder dem größten Wichtungsfaktor und der detektionsseitigen Lage des Funktionsmaximums oder des Schwerpunkts dieser Verwaschungsfunktion verwendet wird.

11. Verfahren nach einem der vorangehenden Ansprüche, **gekennzeichnet durch** der oder die Positionswerte anhand der unmittelbar erhaltenen Verwaschungsfunktionen oder einer zumindest im Bereich der Maxima geglätteten Verwaschungsfunktionen ermittelt werden.

12. Verfahren nach einem der Ansprüche 1 bis 10, **dadurch gekennzeichnet, daß** die erhaltenen Verwaschungsfunktionen zur Bestimmung von Approximationsfunktionen approximiert werden, wobei der oder die Positionswerte anhand der Approximationsfunktionen ermittelt werden.

13. Verfahren nach Anspruch 12, **dadurch gekennzeichnet, daß** die jeweilige Verwaschungsfunktion mittels einer oder mehrerer Gaußfunktionen approximiert wird.

14. Vorrichtung zur Durchführung des Verfahrens nach einem der Ansprüche 1 bis 13, umfassend ein Bestrahlungsmittel (1) ein Detektormittel (3), ein Steuermittel (9) und ein Rechenmittel (10), **dadurch gekennzeichnet, daß** das Rechenmittel (10) zum Ermitteln eines optimalen Untersuchungsortes nach dem Verfahren gemäß einem der Ansprüche 1 bis 13 und das Steuerungsmittel (9) zur Steuerung des Bestrahlungmittels in Abhängigkeit des Ermittlungsergebnisses ausgebildet ist.

15. Vorrichtung nach Anspruch 14, **dadurch gekennzeichnet, daß** das Bestrahlungsmittel (1) eine Strahlungsquelle (2) für eine bestimmte Wellenlänge aufweist und von dem Steuermittel (9) steuerbare Mittel zum Bewegen der Strahlungsquelle vorgesehen sind.

16. Vorrichtung nach Anspruch 14, **dadurch gekennzeichnet, daß** das Bestrahlungsmittel (11) mehrere mittels des Steuermittels (9) separat ansteuerbare Strahlungsquellen (12) für eine bestimmte Wellenlänge aufweist.

17. Vorrichtung nach Anspruch 16, **dadurch gekennzeichnet, daß** über die Steuermittel (9) in Abhängigkeit des Ermittlungsergebnisses steuerbare Mittel zum Bewegen wenigstens einer, vorzugsweise aller Strahlungsquellen (12) vorgesehen sind.

18. Vorrichtung nach Anspruch 16 oder 17, **dadurch gekennzeichnet, daß** das Rastermaß (Abstand) der Strahlungsquellen und der Duchmesser des jeweils emittierten Lichtstrahls in einem Verhältnis von ungefähr 1:1 bis ungefähr 3:1, insbesondere 2:1 stehen.

19. Vorrichtung nach Anspruch 18 und 16, **dadurch gekennzeichnet, daß** das Rastermaß der Strahlungsquellen bei gegebener Bewegbarkeit der Strahlungsquellen ungefähr 250 µm bis 750 µm, insbesondere 500 µm und der Strahldurchmesser ungefähr 125 µm bis 500 µm, insbesondere 250 µm beträgt.

20. Vorrichtung nach Anspruch 18 und 17, **dadurch gekennzeichnet, daß** bei nicht bewegbaren Strahlungsquellen das Rastermaß ungefähr 125 µm bis 500 µm, insbesondere 250 µm, und der Strahldurchmesser 125 µm bis 500 µm, insbesondere 250 µm beträgt.

21. Vorrichtung nach einem der Ansprüche 14 bis 20, **dadurch gekennzeichnet, daß** Strahlungsquellen für mehrere Wellenlängen vorgesehen sind.

## Claims

1. Method for locating an examination location for diaphanoscopic examination of a life form, whereby a region of the life form wherein the optimum examination location is suspected is sequentially transilluminated with a radiation, preferably in a wavelength range of the optical tissue window, for registration of scattered light distributions in the form of location-related spread functions, particularly point spread functions, whereby at least one function-specific, location-related feature of each spread function is determined, based whereon a position value defining the examination location is determined.

2. Method according to Claim 1, **characterised in that** the respective position values of the maximums and/or centres of gravity of the spread functions are employed as a feature.

3. Method according to Claim 2, **characterised in that** the position value defining the examination location is determined by averaging from the position values of the maximums or of the centres of gravity.

4. Method according to one of the preceding claims, **characterised in that** each function-specific, location-related feature is weighted with a weighting factor.

5. Method according to Claim 4, **characterised in that** each weighting factor is determined based on at least one, potentially on further location-specific features.

6. Method according to Claim 5, **characterised in that** each weighting factor is multiplicatively determined from the overall radiation density and the standard deviation of each spread function.

7. Method according to one of Claims 4 to 6, **characterised in that** the weighting factors are normed to the highest identified weighting factor.

8. Method according to one of the preceding claims, **characterised in that** a back-transformation into the irradiation plane ensues after determination of the position value.

9. Method according to Claim 8, **characterised in that**, for back-transformation, a further position value is determined, by which the identified first position value relating to the detection plane is shifted.

10. Method according to Claim 9, **characterised in that** the offset between the irradiation-side irradiation location of the spread function with the highest overall irradiation intensity or the highest weighting factor and the detection-side position of the function maximum or of the centre of gravity of this spread function is employed as a further position value.

11. Method according to one of the preceding claims, **characterised by** the position value or position values being determined on the basis of the directly obtained spread functions or a spread functions [sic] smoothed at least in the region of the maximums,

12. Method according to one of the Claims 1 to 10, **characterised in that** the spread functions that are obtained are approximated for the determination of approximation functions, whereby the position value or values are determined on the basis of the approximation functions.

13. Method according to Claim 12, **characterised in that** the respective spread function is approximated with one or more Gaussian functions.

14. Apparatus for the implementation of the method according to one of Claims 1 to 13, comprising an irradiation means (1), a detector means (3), a control means (9) and a computer means (10), **characterised in that** the computer means (10) is fashioned for determining an optimum examination location according to the method according to one of Claims 1 to 13, and the control means (9) is fashioned for the control of the irradiation means dependant on the determination result.

15. Apparatus according to Claim 14, **characterised in that** the irradiation means (1) comprises a radiation source (2) for a specific wavelength, and means controllable by the control means (9) are provided for moving the radiation source.

16. Apparatus according to Claim 14, **characterised in that** the irradiation means (11) comprises a plurality of radiation sources (12) for a specific wavelength that can be separately driven with the control means (9).

17. Apparatus according to Claim 16, **characterised in that** means controllable via the control means (9) dependent on the determination result are provided for moving at least one, preferably all radiation sources (12).

18. Apparatus according to Claim 16 or 17, **characterised in that** the grid dimension (spacing) of the radiation sources and the diameter of the respectively emitted light beam reside in a ratio of approximately 1: 1 to approximately 3:1, particularly 2:1.

19. Apparatus according to Claim 18 and 16, **characterised in that** the grid dimension of the radiation sources at a given mobility of the radiation sources amounts to approximately 250 µm to 750 µm, particularly 500 µm, and the beam diameter amounts to approximately 125 µm to 500 µm, particularly 250 µm.

20. Apparatus according to Claim 18 and 17, **characterised in that**, given immobile radiation sources, the grid dimension amounts to approximately 125 µm to 500 µm, particularly 250 µm, and the beam diameter amounts to 125 µm to 500 µm, particularly 250 µm.

21. Apparatus according to one of Claims 14 to 20, **characterised in that** radiation sources for a plurality of wavelengths are provided.

## Revendications

1. Procédé qui est destiné à trouver un point d'examen pour l'examen diaphanoscopique d'un être vivant, dans lequel une zone de l'être vivant dans laquelle le point d'examen optimal est supposé être est traversée séquentiellement avec un rayonnement de préférence dans une plage de longueur d'onde de la fenêtre optique du tissu pour l'enregistrement de distributions de lumière diffusée sous la forme de fonctions de flou locales, notamment de fonctions de flou ponctuelles, et dans lequel on détermine au moins une caractéristique locale de chaque fonction de flou, laquelle caractéristique locale est spécifique à la fonction et sur la base de laquelle on détermine une valeur de position définissant le point d'examen.

2. Procédé selon la revendication 1, **caractérisé par le fait qu'**on utilise comme caractéristique les valeurs de position respectives des maxima et/ou des centres de gravité des fonctions de flou.

3. Procédé selon la revendication 2, **caractérisé par le fait qu'**on détermine la valeur de position définissant le point d'examen en formant une moyenne à partir des valeurs de position des maxima ou des centres de gravité.

4. Procédé selon l'une des revendications précédentes, **caractérisé par le fait qu'**on pondère chaque caractéristique locale spécifique à la fonction avec un facteur de pondération.

5. Procédé selon la revendication 4, **caractérisé par le fait qu'**on détermine chaque facteur de pondération en se basant sur au moins une, éventuellement autre, caractéristique spécifique à la fonction.

6. Procédé selon la revendication 5, **caractérisé par le fait qu'**on détermine chaque facteur de pondération par multiplication à partir de la densité de rayonnement totale et de l'écart type de chaque fonction de flou.

7. Procédé selon l'une des revendications 4 à 6, **caractérisé par le fait qu'**on norme les facteurs de pondération sur le plus grand facteur de pondération déterminé.

8. Procédé selon l'une des revendications précédentes, **caractérisé par le fait que**, après la détermination de la valeur de position, on effectue une transformation inverse vers le plan de radiation incidente.

9. Procédé selon la revendication 8, **caractérisé par le fait que**, pour la transformation inverse, on détermine une autre valeur de position de laquelle est décalée la première valeur de position déterminée se rapportant au plan de détection.

10. Procédé selon la revendication 9, **caractérisé par le fait que**, comme autre valeur de position, on utilise le décalage entre le point de radiation incidente, du côté de la radiation incidente, de la fonction de flou ayant la plus grande intensité de rayonnement totale ou le plus grand facteur de pondération et la position, du côté de la détection, du maximum ou du centre de gravité de cette fonction de flou.

11. Procédé selon l'une des revendications précédentes, **caractérisé par le fait qu'**on détermine la ou les valeurs de position à l'aide des fonctions de flou directement obtenues ou de l'une des fonctions de flou lissées au moins dans la zone des maxima.

12. Procédé selon l'une des revendications 1 à 10, **caractérisé par le fait qu'**on approche les fonctions de flou obtenues pour déterminer des fonctions approchées, la ou les valeurs de position étant déterminées à l'aide des fonctions approchées.

13. Procédé selon la revendication 12, **caractérisé par le fait qu'**on approche la fonction de flou respective au moyen d'une ou plusieurs fonctions de Gauss.

14. Dispositif pour la mise en oeuvre du procédé selon l'une des revendications 1 à 13, comprenant un moyen d'irradiation (1), un moyen de détection (3), un moyen de commande (9) et un moyen de calcul (10), **caractérisé par le fait que** le moyen de calcul (10) est conçu pour déterminer un point d'examen optimal selon le procédé selon l'une des revendications 1 à 13 et que le moyen de commande (9) est conçu pour commander le moyen d'irradiation en fonction du résultat de détermination.

15. Dispositif selon la revendication 14, **caractérisé par le fait que** le moyen d'irradiation (1) comporte une source de rayonnement (2) pour une certaine longueur d'onde et que des moyens commandables par le moyen de commande (9) sont prévus pour déplacer la source de rayonnement.

16. Dispositif selon la revendication 14, **caractérisé par le fait que** le moyen d'irradiation (11) comporte plusieurs sources de rayonnement (12), commandables séparément par le moyen de commande (9), pour une certaine longueur d'onde.

17. Dispositif selon la revendication 16, **caractérisé par le fait que** des moyens commandables par le moyen de commande (9) en fonction du résultat de détermination sont prévus pour déplacer au moins l'une et de préférence toutes les sources de rayonnement (12).

18. Dispositif selon la revendication 16 ou 17, **caractérisé par le fait que** la distance (écartement) entre sources de rayonnement et le diamètre du faisceau lumineux respectivement émis sont dans un rapport de 1 : 1 environ à 3 : 1 environ, notamment 2: 1.

19. Dispositif selon les revendications 18 et 16, **caractérisé par le fait que** la distance entre sources de rayonnement pour une mobilité donnée des sources de rayonnement vaut environ 250 µm à 750 µm, notamment 500 µm, et le diamètre de faisceau environ 125 µm à 500 µm, notamment 250 µm.

20. Dispositif selon les revendications 18 et 17, **caractérisé par le fait que**, dans le cas de sources de rayonnements immobiles, la distance entre sources de rayonnement vaut environ 125 µm à 500 µm, notamment 250 µm, et le diamètre de faisceau environ 125 µm à 500 µm, notamment 250 µm.

21. Dispositif selon l'une des revendications 14 à 20, **caractérisé par le fait qu'**il est prévu des sources de rayonnement pour plusieurs longueurs d'onde.
